# EUROPEAN PATENT APPLICATION

(11) **EP 4 442 802 A1**
(43) Date of publication of application: **09.10.2024**
(21) Application number: 21965926.5
(22) Date of filing: 30.11.2021
(51) Int. Cl.: C12M 1/16, C12M 1/00, G01N 21/65

(54) **DETECTION CHIP AND PREPARATION METHOD THEREFOR**

(71) Applicant: BGI Shenzhen, Shenzhen, Guangdong 518083 (CN)
(72) Inventor: ZHOU, Fan, Shenzhen, Guangdong 518083 (CN); XU, Xun, Shenzhen, Guangdong 518083 (CN); NI, Jielei, Shenzhen, Guangdong 518083 (CN); SHEN, Mengzhe, Shenzhen, Guangdong 518083 (CN); WANG, Wenbing, Shenzhen, Guangdong 518083 (CN)
(74) Representative: Murgitroyd & Company
(86) International application number: PCT/CN2021/134543
(87) International publication number: WO 2023/097487

(57) **Abstract**

The present application provides a detection chip and a preparation method therefor. The detection chip comprises a substrate material layer; a feature array comprising a plurality of feature units distributed on the substrate material layer; and a feature array protection structure formed on the base material layer, the feature array being distributed in the feature array protection structure, wherein the feature array protection structure comprises at least an adsorption material for adsorbing a biological sample. According to the present application, the excitation power enhanced by the detection chip is improved by at least a plurality of orders of magnitude compared with the incident laser power, and with the design optimization and process improvement of the feature array, the enhancement proportion can be further improved, such that the excitation power received by the fluorescent dye is improved without changing the power of the laser, and stronger fluorescence signal intensity is generated. Therefore, the exposure time can be effectively shortened in a case that the imaging quality and the laser power are not changed, and the sequencing flux is improved.

## Description

### TECHNICAL FIELD

The application relates to the field of the testing of biological samples, specifically relates to a detection chip for biological samples, and a preparation method therefor.

### BACKGROUND

Currently, the fluorescence signal intensity of biological sample detection chips, such as sequencing chips, is directly proportional to the laser power and the quantum yield of the fluorescent dye, as well as the exposure time. When the type of fluorescent dye is fixed, increasing the sequencing throughput is typically achieved by reducing exposure time, while maintaining the same image quality, meaning that the fluorescence signal intensity must remain constant. Therefore, it becomes necessary to increase the power of the laser, which introduces several disadvantages: first, the cost of high-power lasers increases exponentially; second, there is a need to customize optical components with higher damage thresholds, which complicates manufacturing and increases costs; third, when thermal effects reach a certain level, they can affect the quality of the objective lens imaging, leading to decreased sequencing accuracy; fourth, the laser background noise increases and the signal-to-noise ratio drops, necessitating the use of cameras with higher quantum efficiency, which also increases costs. These disadvantages constrain the increase in sequencing system throughput and the reduction of hardware costs, thus necessitating the exploration of alternative approaches for breakthroughs.

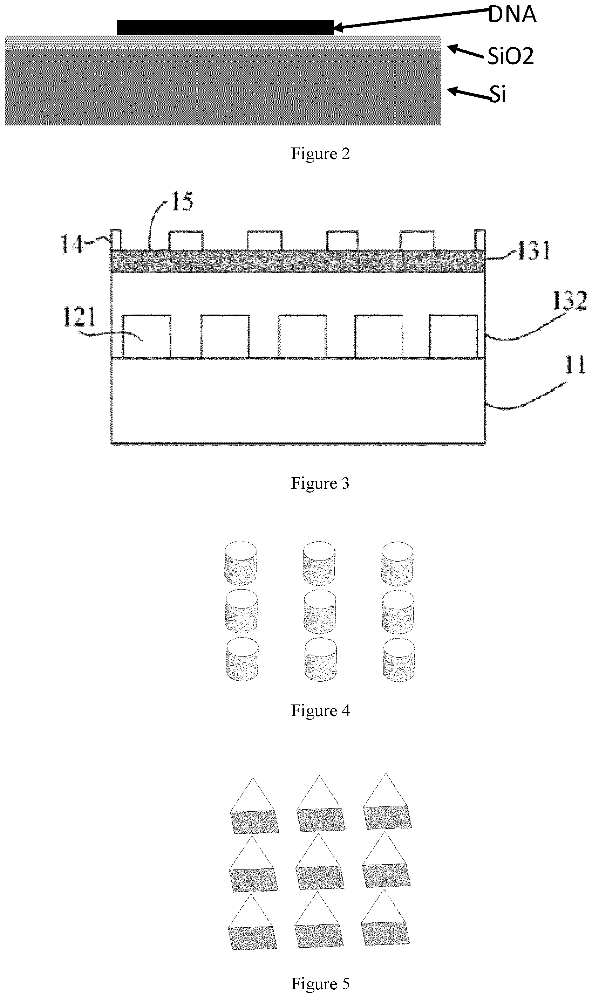

The basic structure of the sequencing chip is shown in Figure 2, with silicon (Si) as the substrate and DNA (deoxyribonucleic acid) connected to an SiO₂ layer. When a laser illuminates a sequencing chip, a portion of the laser passes through the DNA and irradiates onto the substrate; only a portion of this light is reflected back and re-irradiated onto the DNA. Therefore, the actual laser power absorbed by the fluorescent dye on the DNA is less than the incident laser power. Some sequencing chips use a glass substrate, which has a higher transmittance. The light that passes through the DNA on these chips is not reflected back, resulting in an even lower utilization rate of the laser power.

Currently, some technologies attempt to increase the utilization rate of laser power by adding a reflective coating to the substrate. However, this does not effectively enhance the laser power further. As sequencing technology advances and the demand for lower sequencing costs continues, there is a need to increase sequencing throughput and reduce hardware costs. For traditional sequencing chips, achieving higher sequencing throughput and lower hardware costs simultaneously is challenging. Increasing throughput means requiring higher-power lasers, optical components with higher damage thresholds, and more sensitive detectors, which significantly raise hardware costs.

### CONTENT OF THE PRESENT INVENTION

The application primarily aims to provide a detection chip and a preparation method therefor to improve on the above deficiencies in the prior art.

The application addresses the above technical issues by the following technical solutions:

As one aspect of the present disclosure, a detection chip is provided, comprising:
a substrate material layer;
a feature array comprising a plurality of feature units distributed on the substrate material layer; and
a feature array protection structure formed on the substrate material layer, the feature array being distributed within the feature array protection structure, wherein the feature array protection structure comprises at least an adsorption material for adsorbing a biological sample.

As an optional embodiment, the plurality of feature units are regularly distributed on the substrate material layer.

As an optional embodiment, the plurality of feature units are periodically and regularly distributed on the substrate material layer.

As an optional embodiment, the detection chip further comprises a sidewall material layer formed on the feature array protection structure;
the sidewall material layer comprises a plurality of sidewall materials, wherein the positions of sample wells formed on the feature array protection structure by adjacent sidewall materials correspond one-to-one with those of the feature units along the extension direction of the feature units, and the sample wells are used to accommodate the biological samples.

As an optional embodiment, the feature array protection structure comprises a feature array protection layer and an adsorption material layer formed by the adsorption material;
the feature array protection structure is formed on the substrate material layer, the feature array is distributed within the feature array protection layer, the adsorption material layer is formed on the feature array protection layer, and the sidewall material layer is formed on the adsorption material layer.

As an optional embodiment, the feature array protection layer comprises a first feature array protection layer and a second feature array protection layer;
the feature array is distributed within the first feature array protection layer, and the second feature array protection layer is formed on the first feature array protection layer and is in contact with the lower surface of the adsorption material layer.

As an optional embodiment, the thickness of the feature array protection layer ranges from 5 nm to 200 nm; and/or,
the thickness of the adsorption material layer ranges from 5 nm to 200 nm; and/or,
the thickness of the sidewall material layer ranges from 10 nm to 1000 nm.

As an optional embodiment, the material for the feature array protection layer includes one or more than one of silicon dioxide (SiO₂), silicon nitride (Si₃N₄), and titanium dioxide (TiO₂); and/or,
the material for the sidewall material layer includes one or more than one of SiO₂ and Si₃N₄.

As an optional embodiment, the feature array protection structure comprises an adsorption material layer formed by the adsorption material;
the adsorption material layer is formed on the substrate material layer, the feature array is distributed within the adsorption material layer, and the sidewall material layer is formed on the adsorption material layer.

As an optional embodiment, the adsorption material layer comprises a first adsorption material layer and a second adsorption material layer;
the feature array is distributed within the first adsorption material layer, and the second adsorption material layer is formed on the first adsorption layer and is in contact with the lower surface of the sidewall material layer.

As an optional embodiment, the feature array protection structure comprises a feature array protection layer and a sample adsorption layer formed by the adsorption material;
the feature array protection layer is formed on the substrate material layer, the feature array is distributed within the feature array protection layer, and the sample adsorption layer is formed on the feature array protection layer;
specifically, the sample adsorption layer comprises a plurality of passivation areas and a plurality of amination areas, wherein the positions of the amination areas correspond one-to-one with those of the feature units along the extension direction of the feature units, and the amination areas are used for adsorbing the biological samples.

As an optional embodiment, the feature array protection layer comprises a first feature array protection layer and a second feature array protection layer;
the feature array is distributed within the first feature array protection layer, and the second feature array protection layer is formed on the first feature array protection layer and is in contact with the lower surface of the sample adsorption layer.

As an optional embodiment, the material for the substrate material layer includes one or more than one of silicon, quartz, or glass.

As an optional embodiment, the adsorption material includes one or more than one of titanium nitride (TiN), titanium dioxide (TiO₂), silver (Ag), zirconium dioxide (ZrO₂), or zinc oxide (ZnO).

As an optional embodiment, the feature array comprises a metal array, and the feature unit comprises a metal material;

As an optional embodiment, the metal material includes one or more than one of silver (Ag), gold (Au), copper (Cu), or aluminum (Al).

As an optional embodiment, the plurality of feature units are periodically distributed on the substrate material layer in one or more geometrical shapes including polygon, circle, or ellipse; and/or,
the structure of the feature unit comprises a cylindrical or spherical shape; and/or,
the cross-sectional shape of the feature unit includes polygon, circle, or ellipse.

As an optional embodiment, the thickness of the feature unit ranges from 20 nm to 300 nm; and/or,
the cross-sectional dimension of the feature unit ranges from 20 nm to 300 nm; and/or,
the spacing between the centers of two adjacent feature units ranges from 100 nm to 1000 nm.

As an optional embodiment, the top surface of the feature array protection structure is elevated above the top surface of any of the feature units.

As another aspect of the present disclosure, a preparation method for the detection chip is provided, by which the detection chip as described above is prepared;
the preparation method comprises:
providing a substrate material layer;
forming the feature array on the substrate material layer, the feature array comprising a plurality of feature units distributed on the substrate material layer;
forming the feature array protection structure on the substrate material layer, so that the feature array is distributed within the feature array protection structure, wherein the feature array protection structure comprises at least an adsorption material for adsorbing a biological sample.

According to the content of this application, those skilled in the art can understand other aspects of this application

The positive advancements of this application include:

This application introduces a novel biological sample detection chip and a preparation method thereof, based on localized surface plasmon enhancement. The excitation power enhanced by the detection chip is improved by at least six to ten orders of magnitude compared with the incident laser power, and with the design optimization and process improvement of the feature array, the enhancement proportion can be further improved, such that the excitation power received by the fluorescent dye is improved without changing the power of the laser, and stronger fluorescence signal intensity is generated. Therefore, the exposure time can be effectively shortened in a case that the imaging quality and the laser power are not changed, and the sequencing throughput is improved.

### BRIEF DESCRIPTION OF THE DRAWINGS

After reading the detailed description of the embodiments of this application in conjunction with the accompanying drawings, the features and advantages of this application can be better understood. In the drawings, the components are not necessarily drawn to scale, and components with similar related characteristics or features may have the same or similar reference numerals.
Figure 1 is a schematic diagram of a partial structure of a detection chip according to an embodiment of this application.
Figure 2 is a schematic diagram of basic structure of an existing sequencing chip.
Figure 3 is a schematic diagram of a partial structure of a detection chip of first-type structure according to an embodiment of this application.
Figure 4 is a schematic diagram showing the three-dimensional structure of a cylindrical feature unit.
Figure 5 is a schematic diagram showing the three-dimensional structure of a triangular prism-shaped feature unit.
Figure 6 is a schematic diagram showing the three-dimensional structure of a cubic feature unit.
Figure 7 is a schematic diagram showing the three-dimensional structure of a spherical feature unit.
Figure 8 is a structural schematic diagram showing the quadrilateral periodic distribution of cylindrical feature units.
Figure 9 is a structural schematic diagram of the smallest periodic unit of the feature array in Figure 8.
Figure 10 is a structural schematic diagram showing the hexagonal periodic distribution of cylindrical feature units.
Figure 11 is a structural schematic diagram of the smallest periodic unit of the feature array in Figure 10.
Figure 12 is a structural schematic diagram showing the circular periodic distribution of cylindrical feature units.
Figure 13 is a structural schematic diagram of the smallest periodic unit of the feature array in Figure 12.
Figure 14 is a schematic diagram showing the electric field distribution formed on the surface of the feature array.
Figure 15 is a schematic diagram showing the electric field distribution formed between two cylindrical feature units.
Figure 16 is a schematic diagram showing the optical path of the imaging device for a detection chip according to an embodiment of this application.
Figure 17 is a schematic diagram of a partial structure of a detection chip of a second-type structure according to an embodiment of this application.
Figure 18 is a schematic diagram of a partial structure of a detection chip of a third-type structure according to an embodiment of this application.
Figure 19 is a flowchart schematic diagram of a method for preparing a detection chip according to another embodiment of this application.
Figure 20 is a schematic diagram of processing technology of a method for preparing a detection chip according to another embodiment of this application.

### DETAILED DESCRIPTION OF THE PREFERRED EMBODIMENT

The following further illustrates the present application by embodiments, but does not limit it within the scope of the embodiments.

It should be noted that references in the specification to "one embodiment," "an optional embodiment," "another embodiment," etc., indicate that the described embodiments may include specific features, structures, or characteristics, but not every embodiment necessarily includes the particular feature, structure, or characteristic. Moreover, such phrases are not necessarily referring to the same embodiments. Furthermore, when specific features, structures, or characteristics are described in conjunction with the embodiments, it is within the knowledge of those skilled in the relevant fields to implement such features, structures, or characteristics in conjunction with other embodiments whether or not explicitly described.

In the description of the present application, it is understood that terms such as "center," "lateral," "top," "bottom," "left," "right," "vertical," "horizontal," "upper," "lower," "inner," "outer," etc., indicate orientations or positional relationships based on those shown in the figures, these terms are used merely for convenience in describing the application and for simplifying the description, and do not imply that the referred devices or elements must have a specific orientation, be constructed and operated in a specific orientation, therefore these terms should not be construed as limiting the application. Additionally, the terms "first," "second," are used merely for descriptive purposes and should not be interpreted as indicating or implying relative importance, or implying the quantity of the technical features as indicated. Therefore, the features defined as "first," "second" can explicitly or implicitly include one or more such features. In the descriptions of the present application, unless otherwise specified, the term "multiple" means two or more Furthermore, the term "comprise/comprising," and any of its variations, are intended to convey a non-exclusive inclusion.

In the descriptions of the present application, it should be noted that unless explicitly defined and limited, terms such as "installed/mounted," "communicated," and "connected" should be understood in a broad sense, for example, these terms could denote fixed connections or detachable connections, and could be integrally connected; and they might represent mechanical connections or electrical connections; connections could be direct or indirect by an intermediary medium, and could involve internal communications within two components. Those of ordinary skill in the art can understand the specific meanings of these terms in the present application based on the context and specific circumstances.

The terminology used here is intended merely for describing specific embodiments, not be intended to limit the exemplary implementations. Unless explicitly specified otherwise in the context, the singular forms "a," "an," and "one" should also be understood to include the plural form. It should also be understood that the term "comprise/comprising" and/or "include/including," as used herein, specifies the presence of stated features, integers, steps, operations, elements, and/or components, but does not preclude the presence or addition of one or more other features, integers, steps, operations, elements, components, and/or their combinations.

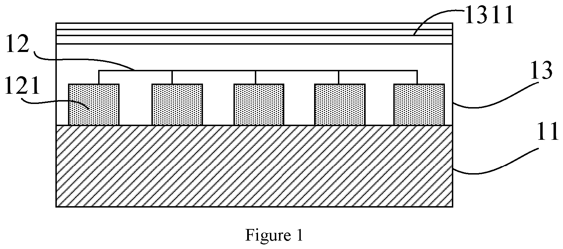

To overcome existing deficiencies, as shown in Figure 1, this embodiment provides a detection chip, which primarily comprises: a substrate material layer 11; a feature array 12 comprising a plurality of feature units 121 distributed on the substrate material layer 11; and a feature array protection structure 13 formed on the substrate material layer 11, the feature array 12 being distributed in the feature array protection structure 13, wherein the feature array protection structure 13 comprises at least an adsorption material 1311 for adsorbing a biological sample.

In this embodiment, the detection chip is preferably a sequencing chip, but it is not limited to sequencing chips and can also be other biological or chemical detection chips. Adjustments or selections can be made according to actual needs or potential needs that may arise. In addition to its application in the sequencing field, this detection chip can also be applied in various other fields such as protein analysis, single-cell analysis, and drug screening. Based on achieving the functionalities mentioned above, adjustments or selections can be made in conjunction with actual or potential needs.

In this embodiment, the biological samples can, for example, include DNA nanoball, quantum dots, and fluorescent nanoball in sequencers, among others. However, the type of sample is not specifically limited in this embodiment; it can be set and adjusted according to actual needs and scenarios, or potential needs and scenarios that may arise.

In this embodiment, the top surface of the feature array protection structure 13 is at least elevated above the top surface of any feature unit 121, ensuring that the feature array protection structure 13 effectively shields each feature unit 121.

This embodiment provides a novel biological sample detection chip based on localized surface plasmon resonance enhancement. The excitation power enhanced by the detection chip is improved by at least six to ten orders of magnitude compared with the incident laser power, and with the design optimization and process improvement of the feature array, the enhancement proportion can be further improved, such that the excitation power received by the fluorescent dye is improved without changing the power of the laser, and stronger fluorescence signal intensity is generated. Therefore, the exposure time can be effectively shortened in a case that the imaging quality and the laser power are not changed, and the sequencing throughput is improved.

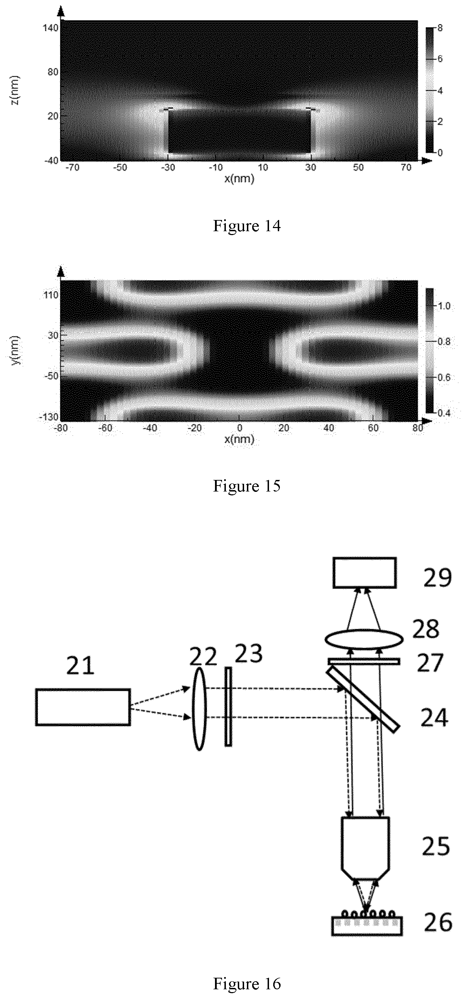

In this embodiment, the surface of the detection chip employs a nanostructured feature array that generates a localized surface plasmon resonance effect when illuminated by a laser, thereby enhancing the excitation power. As shown in Figure 16, the figure 16 illustrates the optical path schematic diagram when the imaging device of the detection chip emits a laser. Specifically, the laser 21 passes through a collimating lens 22, transforming it into parallel light that irradiates onto the excitation filter 23. The filtered laser light then irradiates onto a dichroic mirror 24. Subsequently, the laser is reflected to the back end of the objective lens 25 and focused by the objective lens 25 to irradiate onto the chip assembly 26, which includes the detection chip provided in this embodiment. The nanostructured feature array on the detection chip generates localized surface plasmons, and the fluorescent dye of DNA on the chip emits a fluorescence signal. The fluorescence signal is collected by the objective lens 25, passes through the dichroic mirror 24, and irradiates onto the emission filter 27. Finally, the signal is focused by lens 28 onto the detector 29 to produce the sequencing image.

Localized surface plasmon enhancement refers to a phenomenon where, upon laser is irradiated on the nanostructured feature unit, if the frequency of the laser is the same as the vibrational frequency of the nanostructured feature units, these units strongly absorb the light, leading to localized surface plasmon resonance and forming an electric field distribution on the surface of the nanostructured feature units, as illustrated in Figure 14. The electric field intensity at the sharp edges of the nanostructured feature units can increase by three to four orders of magnitude compared to the incident light. For certain specially designed nanostructured feature array structures, such as between two cylindrical bodies as shown in Figure 15, the electric field intensity can be enhanced by six to ten orders of magnitude.

The resonance frequency of nanostructured feature units is closely related to factors such as the density of electrons, the effective mass of electrons, and the shape and size of the charge distribution. Therefore, the localized surface plasmon resonance effect can be tuned by adjusting the material, size, shape, and surrounding medium of the nanostructured feature units. A common approach involves using optical simulation software such as FDTD (Finite Difference Time Domain) to simulate and calculate structural parameters, and then processing corresponding detection chips. After real-world testing, feedback is provided to the simulation model, which is then adjusted to further optimize and determine the best structural parameters for achieving maximum power enhancement effects.

Specifically, as an optional embodiment, as shown in Figure 3, the detection chip of the first-type structure primarily comprises: a substrate material layer 11, a feature array that comprises a plurality of feature units 121 regularly distributed on the substrate material layer 11, a feature array protection structure, and a sidewall material layer formed on the feature array protection structure.

Among them, the feature array protection structure comprises a feature array protection layer 132 and an adsorption material layer 131 formed by adsorption material. The feature array protection layer 132 is formed on the substrate material layer 11, the feature array is distributed within the feature array protection layer 132, the adsorption material layer 131 is formed on the feature array protection layer 132, and the sidewall material layer is formed on the adsorption material layer 131. In this embodiment, the feature array protection layer 132 can be further divided into a first feature array protection layer and a second feature array protection layer (not specifically distinguished in the figure). The feature array is distributed within the first feature array protection layer (which may have a thickness similar to that of the feature units 121), and the second feature array protection layer is formed on the first feature array protection layer and is in contact of the lower surface of the adsorption material layer 131. The sidewall material layer comprises a plurality of sidewall materials 14, wherein the positions of sample wells 15 formed on the adsorption material layer 131 by adjacent sidewall materials 14 correspond one-to-one with those of the feature units 121 along the extension direction of the feature units 121, and the sample wells 15 are used to accommodate the biological samples.

The materials for the first feature array protection layer and the second feature array protection layer can be the same or different. The adsorption material layer 131 can be made from the same or different materials as those used in the first feature array protection layer and the second feature array protection layer. The sidewall material layer can also be made from the same or different materials as those used in the first feature array protection layer and the second feature array protection layer. Therefore, adjustments and selections can be made according to actual needs or potential requirements that may arise.

As an optional embodiment, the material for the substrate material layer 11 includes one or more than one of silicon, quartz, or glass, but are not limited to them. Adjustments and selections can be made according to actual needs or potential requirements that may arise.

As an optional embodiment, the feature unit 121 can be made of metallic materials, meaning that the feature array can be a metal array. But this is not a limitation, and selections and adjustments can be made according to actual needs or potential requirements that may arise.

The metallic materials can include one or more than one of Ag, Au, Cu, and Al, but are not limited to them. Adjustments and selections can be made according to actual needs or potential requirements that may arise.

As an optional embodiment, the plurality of feature units 121 are distributed on the substrate material layer 11 in one or more geometrical shapes including polygon, circle, or ellipse; the structure of the feature unit 121 comprises a cylindrical or spherical shape; the cross-sectional shape of the feature unit 121 includes polygon, circle, or ellipse. However, these specifications do not constitute limitations, and adjustments and selections can be made according to actual needs or potential requirements that may arise.

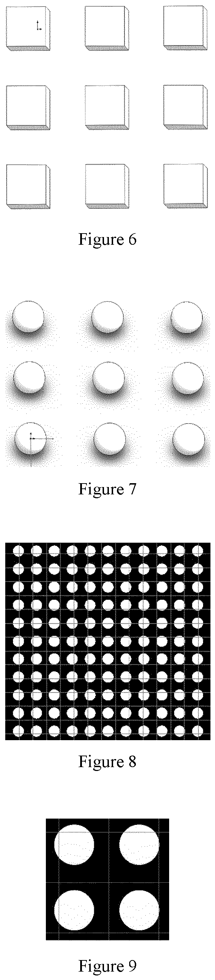

Specifically, regarding the structure and shape of the feature units: as shown in Figure 4, the feature units can be cylindrical in shape; as shown in Figure 5, the feature units can be triangular prisms; as shown in Figure 6, the feature units can be cubic in shape; as shown in Figure 7, the feature units can be spherical in shape.

As an optional embodiment, the plurality of feature units 121 are periodically and regularly distributed on the substrate material layer 11.

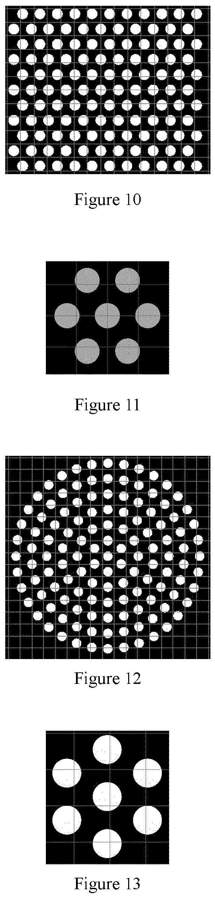

Regarding the periodic and regular distribution of feature units: as shown in Figures 8 and 9, the feature array can be arranged in a quadrilateral periodic distribution; as shown in Figures 10 and 11, the feature array can be arranged in a hexagonal periodic distribution; as shown in Figures 12 and 13, the feature array can be arranged in a circular periodic distribution.

In this embodiment, the thickness and cross-sectional dimensions (such as diameter) of the feature units can influence the localized surface plasmon resonance effect and are subject to the limitations of nanofabrication capabilities. The thickness of the feature unit can range from 20 nm to 300 nm, with a preferred thickness of 60 nm; the cross-sectional dimensions of the feature unit can range from 20 nm to 300 nm, with a preferred cross-sectional dimension of 100 nm. The period, which is the spacing between the centers of adjacent feature units, can be determined based on the optical system's diffraction limit. The spacing between the centers of the adjacent feature units ranges from 100 nm to 1000 nm, preferably set at 700 nm.

As an optional embodiment, the material for the feature array protection layer 132 include one or more than one of SiO₂, Si₃N₄, and TiO₂, but are not limited to them. Adjustments and selections can be made according to actual needs or potential requirements that may arise.

In this embodiment, the thickness of the feature array protection layer 132 ranges from 5 nm to 200 nm, preferably set at 10 nm. A thickness less than 5 nm may not sufficiently protect the metal, whereas a thickness greater than 200 nm could result in lower localized surface plasmon energy.

As an optional embodiment, the adsorption material includes one or more than one of TiN, TiO₂, Ag, ZrO₂, and ZnO, but are not limited to them. Adjustments and selections can be made according to actual needs or potential requirements that may arise.

In this embodiment, the thickness of the adsorption material layer 131 ranges from 5 nm to 200 nm, preferably set at 10 nm. A thickness less than 5 nm may not sufficiently protect the metal, whereas a thickness greater than 200 nm could result in lower localized surface plasmon energy.

As an optional embodiment, the material for the sidewall material layer includes one or more than one of SiO₂ and Si₃N₄, but are not limited to them. Adjustments and selections can be made according to actual needs or potential requirements that may arise.

In this embodiment, the thickness of sidewall material layer ranges from 10 nm to 1000 nm, preferably set at 100 nm. The thickness of the sidewall material is determined based on the size of the biological sample (for example, the diameter of a DNA nanoball).

In this embodiment, various parameters of each layer (such as material, thickness, diameter, pitch (the distance between the centers of two adjacent feature units), shape, and periodic distribution) can be determined using optical simulation software FDTD based on actual usage conditions (laser wavelength, angle of incidence, uniformity of the light spot, fluorescent dyes, etc.). And based on the actual test results of the processing detection chip, feedback is given to the simulation software. After optimizing the simulation again, the optimal detection chip parameters are obtained to achieve maximum excitation power enhancement.

As a specific embodiment, considering the actual usage scenario of this embodiment (532 nm & 640 nm laser wavelengths, 0-degree angle of incidence, imaging area illumination uniformity greater than 95%, fluorescent dyes AF532 & Cy5 (fluorescence dye type)), the preferred detection chip structure is obtained, with the following structures: an 8-inch silicon wafer substrate, a nano-array of Ag cylinders (60 nm height, 130 nm diameter, 700 nm pitch, square periodic distribution), a SiO₂ feature array protection layer (filling the gaps between Ag cylinders and extending 10 nm above the tops of the Ag cylinders), a TiO₂ adsorption material layer (where DNA samples can be adsorbed on TiO₂, with a thickness of 10 nm), and a SiO₂ sidewall material layer (with a sidewall height of 100 nm, the distribution period similar to that of the feature array).

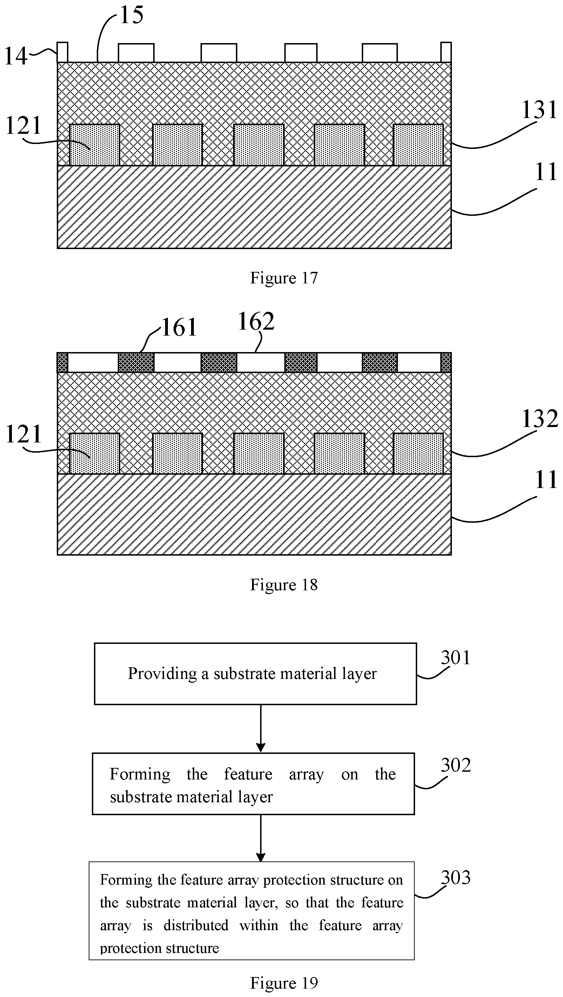

As another optional embodiment, as shown in Figure 17, the detection chip of the second-type structure primarily comprises: a substrate material layer 11, a feature array that comprises a plurality of feature units 121 distributed on the substrate material layer 11, a feature array protection structure, and a sidewall material layer formed on the feature array protection structure.

The feature array protection structure comprises an adsorption material layer 131 formed by adsorption materials. The adsorption material layer 131 is formed on the substrate material layer 11, the feature array is distributed within the adsorption material layer 131, and the sidewall material layer is formed on the adsorption material layer 131.

In this embodiment, the adsorption material layer 131 can be further divided into a first adsorption material layer and a second adsorption material layer (not specifically distinguished in the figure). The feature array is distributed within the first adsorption material layer (which may have a thickness similar to that of the feature units 121), and the second adsorption material layer is formed on the first adsorption material layer and is in contact of the lower surface of the sidewall material layer.

The sidewall material layer comprises a plurality of sidewall materials 14, wherein the positions of sample wells 15 formed on the adsorption material layer 131 by adjacent sidewall materials 14 correspond one-to-one with those of the feature units 121 along the extension direction of the feature units 121, and the sample wells 15 are used to accommodate the biological samples.

The materials for the first adsorption material layer and the second adsorption material layer can be the same or different. The sidewall material layer can also be made from the same or different materials as those used in the first adsorption material layer and the second adsorption material layer. Therefore, selections and adjustments can be made according to actual needs or potential requirements that may arise.

In this embodiment, the detection chip of the second-type structure is similar in materials, dimensions, and the distribution and shape of the feature array to the detection chip of the first-type structure described previously. Therefore, the specific parameters of the detection chip of the first-type structure can be referred to without restating them in detail here.

In this embodiment, in the detection chip of the second-type structure, the feature array protection layer and the adsorption material layer are combined, and only the adsorption material layer is used to achieve the effect of both sample adsorption and protection of the feature array, thereby simplifying the structure. Moreover, since the intensity of the localized surface plasmon is related to the distance between the feature arrays; the shorter this distance, the stronger the enhancement effect. Thus, by merging the feature array protection layer with the adsorption material layer, the distance between the biological sample's fluorescent dyes and the feature array is effectively reduced.

As another optional embodiment, as shown in Figure 18, the detection chip of the third-type structure primarily comprises: a substrate material layer 11, a feature array that comprises a plurality of feature units 121 distributed on the substrate material layer 11, and a feature array protection structure.

The feature array protection structure comprises a feature array protection layer 132 and a sample adsorption layer formed by adsorption materials. The feature array protection structure 132 is formed on the substrate material layer 11, the feature array is distributed within the feature array protection layer 132, and the sample adsorption layer is formed on the feature array protection layer 132.

The sample adsorption layer specifically comprises a plurality of passivation areas 161 and several amination areas 162, wherein the positions of the amination areas 162 correspond one-to-one with those of the feature units along the extension direction of the feature units, and the amination areas 162 are used for adsorbing the biological samples.

As an optional embodiment, the passivation area 161 can be formed by treatment with the chemical reagent hexamethyldisilazane (HMDS). The amination area 162 is a region with amino groups, which can be used to connect DNA and others. The specific processes for forming the passivation area 161 using HMDS treatment and for creating the amination area 162 can refer to existing processing techniques, and thus are not detailed here.

In this embodiment, the feature array protection layer 132 can be further divided into a first feature array protection layer and a second feature array protection layer (not specifically distinguished in the figure). The feature array is distributed within the first feature array protection layer (which may have a thickness similar to that of the feature units 121), and the second feature array protection layer is formed on the first feature array protection layer and is in contact of the lower surface of the adsorption material layer 131.

The materials for the first feature array protection layer and the second feature array protection layer can be the same or different. Therefore, selections and adjustments can be made according to actual needs or potential requirements that may arise.

In this embodiment, the detection chip of the third-type structure is similar in materials, dimensions, and the distribution and shape of the feature array to the detection chip of the first-type structure described previously. Therefore, the specific parameters of the detection chip of the first-type structure can be referred to without restating them in detail here.

In this embodiment, the detection chip of the third-type structure does not require the formation of a sidewall material layer. The use of the amination area formed on the feature array protection layer is sufficient for adsorbing biological samples, which simplifies the overall structure of the detection chip. As a result, it effectively streamlines the fabrication process of the detection chip, enhances manufacturing efficiency, and reduces production costs. The detection chip provided in this embodiment offers the following beneficial effects:
1. This embodiment can reduce system hardware costs: by using the detection chip of this embodiment, which incorporates localized surface plasmon enhancement technology, the excitation power for the fluorescent dyes on DNA is significantly increased, thus yielding a higher fluorescence signal. Additionally, as the energy of localized surface plasmons decays exponentially with distance, and the laser background is low, the signal-to-noise ratio of imaging images is effectively improved. Consequently, for the same image quality, it is possible to use lower-power lasers, less expensive components, and detectors with lower quantum efficiency and sensitivity, substantially reducing the overall hardware costs.
2. This embodiment can increase sequencing throughput: for the same hardware system, since the detection chip in this embodiment provides higher fluorescence signals and a better signal-to-noise ratio, it allows for reduced exposure times while maintaining the same imaging quality. This reduction in exposure time decreases the duration of each sequencing run, thereby enhancing the overall sequencing throughput.
3. This embodiment can overcome the current throughput bottlenecks of traditional sequencing chips: to increase sequencing throughput, sequencers using traditional sequencing chips need to continually reduce the duration of each run. Common methods include shortening exposure times and the movement times of the platform, which necessitates the use of faster and more advanced hardware. However, there are limits to hardware improvements, thus creating bottlenecks in throughput enhancement. The novel detection chip provided by this embodiment can break through these bottlenecks of traditional sequencing chips.

As another embodiment, as shown in Figure 19, this embodiment provides a preparation method for the detection chip as described in the above embodiment, and the preparation method primarily comprises the following steps:
step 301: providing a substrate material layer;
step 302: forming a feature array on the substrate material layer;
step 303: forming the feature array protection structure on the substrate material layer, so that the feature array is distributed within the feature array protection structure.

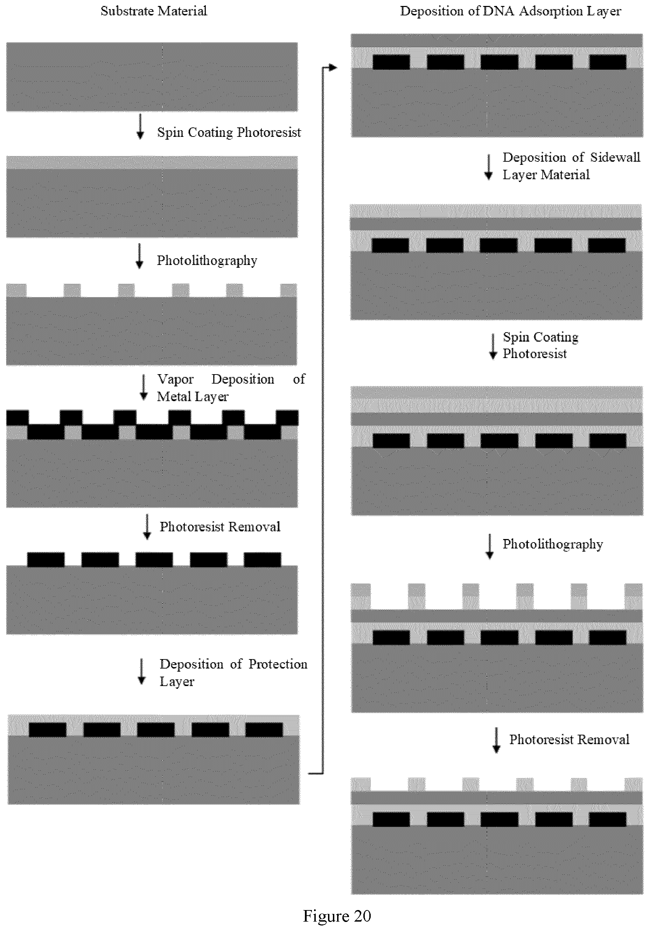

Specifically, as a particular embodiment, as shown in Figure 20, a 300 nm thick layer of ultraviolet photoresist is spin-coated onto an 8-inch silicon wafer. The photoresist is then exposed and developed to create the feature array pattern. Next, a layer of metal (such as Ag) is deposited. Excess photoresist and metal are then removed using acetone to form a layer of Ag cylinders as the feature array. Subsequently, a layer of SiO₂ is deposited via electron beam evaporation to serve as the feature array protective layer, filling the gaps between the Ag cylinders and extending 10 nm above the tops of the Ag cylinders. A 10 nm thick layer of TiO₂ (acting as the adsorption material layer) and a 100 nm thick layer of SiO₂ (acting as the sidewall material layer) are then deposited via electron beam evaporation. A 300 nm thick layer of photoresist is spin-coated onto it, exposed and developed. The SiO₂ layer is then dry etched to produce the final detection chip.

In this embodiment, the specific fabrication processes and parameters are not strictly defined; they can be finely adjusted based on the actual conditions of the manufacturing equipment.

Using the preparation method for the detection chip provided by this embodiment, a detection chip as described above can be fabricated, which possesses the beneficial effects mentioned above.

Although specific embodiments of the present application have been described above, it should be understood by those skilled in the art that these descriptions are merely illustrative. The scope of the present application is defined by the appended claims. Those skilled in the art can make various changes or modifications to these embodiments without departing from the principles and essence of the present application, and these changes and modifications are intended to fall in the scope of protection of the present application.

## Claims

1. A detection chip, wherein the detection chip comprises:
a substrate material layer;
a feature array comprising a plurality of feature units distributed on the substrate material layer; and
a feature array protection structure formed on the substrate material layer, with the feature array being distributed within the feature array protection structure, wherein the feature array protection structure comprises at least an adsorption material for adsorbing a biological sample.

2. The detection chip as claimed in claim 1, the plurality of feature units are regularly distributed on the substrate material layer.

3. The detection chip as claimed in claim 2, the plurality of feature units are periodically and regularly distributed on the substrate material layer.

4. The detection chip as claimed in claim 1, the detection chip further comprises a sidewall material layer formed on the feature array protection structure;
the sidewall material layer comprises a plurality of sidewall materials, wherein positions of sample wells formed on the feature array protection structure by adjacent sidewall materials correspond one-to-one with those of the feature units along an extension direction of the feature units, and the sample wells are used to accommodate the biological samples.

5. The detection chip as claimed in claim 4, the feature array protection structure comprises a feature array protection layer and an adsorption material layer formed by the adsorption material;
the feature array protection layer is formed on the substrate material layer, the feature array is distributed within the feature array protection layer, the adsorption material layer is formed on the feature array protection layer, and the sidewall material layer is formed on the adsorption material layer.

6. The detection chip as claimed in claim 5, the feature array protection layer comprises a first feature array protection layer and a second feature array protection layer;
the feature array is distributed within the first feature array protection layer, and the second feature array protection layer is formed on the first feature array protection layer and is in contact with a lower surface of the adsorption material layer.

7. The detection chip as claimed in claim 5, the thickness of the feature array protection layer ranges from 5 nm to 200 nm; and/or,
the thickness of the adsorption material layer ranges from 5 nm to 200 nm; and/or,
the thickness of the sidewall material layer ranges from 10 nm to 1000 nm.

8. The detection chip as claimed in claim 5, a material for the feature array protection layer includes one or more than one of SiO₂, Si₃N₄, and TiO₂; and/or,
a material for the sidewall material layer includes one or more than one of SiO₂ and Si₃N₄.

9. The detection chip as claimed in claim 4, the feature array protection structure comprises an adsorption material layer formed by the adsorption material;
the adsorption material layer is formed on the substrate material layer, the feature array is distributed within the adsorption material layer, and the sidewall material layer is formed on the adsorption material layer.

10. The detection chip as claimed in claim 9, the adsorption material layer comprises a first adsorption material layer and a second adsorption material layer;
the feature array is distributed within the first adsorption material layer, and the second adsorption material layer is formed on the first adsorption layer and is in contact with a lower surface of the sidewall material layer.

11. The detection chip as claimed in claim 1, the feature array protection structure comprises a feature array protection layer and a sample adsorption layer formed by the adsorption material;
the feature array protection layer is formed on the substrate material layer, the feature array is distributed within the feature array protection layer, and the sample adsorption layer is formed on the feature array protection layer;
wherein the sample adsorption layer comprises a plurality of passivation areas and a plurality of amination areas, the positions of the amination areas correspond one-to-one with those of the feature units along an extension direction of the feature units, and the amination areas are used for adsorbing the biological samples.

12. The detection chip as claimed in claim 11, the feature array protection layer comprises a first feature array protection layer and a second feature array protection layer;
the feature array is distributed within the first feature array protection layer, and the second feature array protection layer is formed on the first feature array protection layer and is in contact with a lower surface of the sample adsorption layer.

13. The detection chip as claimed in claim 1, the material for the substrate material layer includes one or more than one of silicon, quartz, or glass.

14. The detection chip as claimed in claim 1, the adsorption material includes one or more than one of TiN, TiO₂, Ag, ZrO₂, and ZnO.

15. The detection chip as claimed in any one of claims 1 to 14, the feature array comprises a metal array, and the feature unit comprises a metallic material.

16. The detection chip as claimed in claim 15, the metallic material includes one or more than one of Ag, Au, Cu, and Al.

17. The detection chip as claimed in any one of claims 1 to 14, the plurality of feature units are periodically distributed on the substrate material layer in one or more shapes of polygon, circle, or ellipse; and/or,
a structure of the feature unit comprises a cylindrical or spherical shape; and/or,
a cross-sectional shape of the feature unit includes polygon, circle, or ellipse.

18. The detection chip as claimed in any one of claims 1 to 14, a thickness of the feature unit ranges from 20 nm to 300 nm; and/or,
a cross-sectional dimension of the feature unit ranges from 20 nm to 300 nm; and/or,
a spacing between the centers of two adjacent feature units ranges from 100 nm to 1000 nm.

19. The detection chip as claimed in any one of claims 1 to 14, wherein a top surface of the feature array protection structure is elevated above a top surface of any of the feature units.

20. A preparation method for a detection chip, wherein the detection chip as claimed in any one of claims 1 to 19 is prepared by the preparation method;
the preparation method comprises:
providing the substrate material layer;
forming the feature array on the substrate material layer, the feature array comprising a plurality of feature units distributed on the substrate material layer;
forming the feature array protection structure on the substrate material layer, so that the feature array is distributed within the feature array protection structure, wherein the feature array protection structure comprises at least an adsorption material for adsorbing a biological sample.
